# EUROPÄISCHE PATENTSCHRIFT

(11) **EP 0 539 989 B1**
(45) Veröffentlichungstag und Bekanntmachung des Hinweises auf die Patenterteilung: **18.06.1997**
(21) Anmeldenummer: 92118515.3
(22) Anmeldetag: 29.10.1992
(51) Int. Cl.: C07C 17/00, C07C 19/08

(54) **Verfahren zur Herstellung von 1,1,1,2,3,3,3-Heptafluorpropan (R 227)**
Process for the preparation of 1,1,1,2,3,3,3-heptafluoropropane (R 227)
Procédé pour préparer 1,1,1,2,3,3,3-heptafluoropropane (R 227)

(30) Priorität: 01.11.1991 DE 4136054
(43) Veröffentlichungstag der Anmeldung: 05.05.1993
(73) Patentinhaber: SOLVAY (Société Anonyme), B-1050 Bruxelles (BE)
(72) Erfinder: Hopp, Peter, Dr., W-6238 Hofheim am Ts. (DE)
(74) Vertreter: Jacques, Philippe

(56) Entgegenhaltungen:
- EP-A- 0 379 793
- WO-A-91/05752

## Beschreibung

Die Erfindung betrifft ein Verfahren zur Herstellung von 1,1,1,2,3,3,3-Heptafluorpropan (R 227) durch Hydrodechlorierung von 2-Chlorheptafluorpropan an einem Katalysator. R 227 ist als Treibgas und in Klimaanlagen einsetzbar.

Die Herstellung von R 227 aus Hexafluorpropen und Fluorwasserstoff mit Hilfe eines Aktivkohle-Kataysators ist bereits bekannt (GB-PS 902 590). Allerdings befinden sich im Produkt ungesättigte, giftige Verbindungen, z.B. unumgesetztes Hexafluorpropen.

Aus WO 91/05752 ist die Herstellung von R 227 durch Cl/H-Aüstaüsch an 2-Chlorheptafluorpropan in einem Nickel-Reaktor bekannt. In der EP-A-0 379 793 wird die Hydrodechlorierung von Chlortetrafluorethan an Hydrierkatalysatoren auf Trägern beschrieben.

Gegenstand der vorliegenden Erfindung ist ein Verfahren zur Hersteilung von 1,1,1,2,3,3,3-Heptafluorpropan, dadurch gekennzeichnet, daß man 2-Chlorheptafluorpropan mit Wasserstoff in einem Molverhältnis von 1:1 bis 1:3 in der Gasphase an einem Palladium, enthaltenden Träger-Katalysator bei Temperaturen von 100 bis 300°C und Drucken von 1 bis 5 bar umsetzt.

Als Trägermaterial sind beispielsweise geeignet Aktivkohle, Lithium-Aluminium-Spinell, Aluminiumoxid; vorzugsweise verwendet man Aktivkohle.

Der Gehalt des Träger-Katalysators an Pd beträgt im allgemeinen 0.2 bis 5 Gew.-%, bezogen auf das Gesamtgewicht des Katalysators.

Die Reaktionstemperatur beträgt vorzugsweise 200 bis 300°C.

Pro Mol 2-Chlorheptafluorpropan werden 1 bis 3 Mol Wasserstoff eingesetzt.

Da es sich beim erfindungsgemäßen Verfahren um eine Hydrodechlorierung unter hydrierenden Bedingungen handelt, entstehen im Unterschied zu dem bekannten Verfahren gemäß GB-PS 902 530 keine ungesättigten Verbindungen, insbesondere keine giftigen.

Die Herstellung des als Ausgangsmaterial eingesetzten 2-Chlorheptafluorpropans ist in Chemical Abstracts (C91-081033) beschrieben.

Das entstehende Gasgemisch wird mit Wasser gewaschen, entsäuert und kondensiert. Eine GC-Analyse zeigt die Zusammensetzung des so gewonnenen Rohgases.

### Beispiel

Ein senkrecht angeordneter, elektrisch beheizter Rohrreaktor aus Stahl (Länge = 100 cm, Durchmesser = 2 cm) wurde mit 200 ml Aktivkohle beschickt, die mit 0,7 Gew.-% Palladium dotiert war.

Der Katalysator wurde 6 Stunden lang bei 250°C unter Durchleiten von 5 l Wasserstoff pro Stunde konditioniert. Danach wurden 0,4 Mol/h CF₃-CCIF-CF₃ und 1 Mol/h Wasserstoff bei 250°C und Normaldruck eindosiert. Die den Reaktor verlassenden Reaktionsgase passierten eine Wasserwäsche mit anschließender Trocknung und Kondensation bei -15°C.
- Umsatz:: 89 %
- Selektivität:: 85 %

## Patentansprüche

1. Verfahren zur Herstellung von 1,1,1,2,3,3,3-Heptafluorpropan, dadurch gekennzeichnet, daß man 2-Chlorheptafluorpropan mit Wasserstoff in einem Molverhältnis von 1:1 bis 1:3 in der Gasphase an einem Palladium, enthaltenden Träger-Katalysator bei Temperaturen von 100 bis 300°C und Drucken von 1 bis 5 bar umsetzt.

2. Verfahren nach Anspruch 1, dadurch gekennzeichnet, daß der Gehalt des Träger-Katalysators an Pd, 0,2 bis 5 Gew.-% beträgt.

## Claims

1. A process for the preparation of 1,1,1,2,3,3,3-heptafluoropropane, characterized that 2-chloroheptafluoropropane is reacted with hydrogen in a molar ratio of from 1:1 to 1:3 in the gas phase on a palladium containing supported catalyst at temperatures of from 100 to 300°C and pressures of from 1 to 5 bar.

2. The process as claimed in claim 1, wherein the Pd content of the supported catalyst is from 0.2 to 5 % by weight.

## Revendications

1. Procédé de préparation du 1,1,1,2,3,3,3-heptafluoropropane, caractérisé en ce que l'on fait réagir du 2-chloroheptafluoropropane avec de l'hydrogène dans un rapport molaire allant de 1:1 à 1:3, en phase gazeuse, sur un catalyseur-support contenant du palladium, à des températures allant de 100 jusque 300°C et à des pressions allant de 1 jusque 5 bar.

2. Procédé selon la revendication 1, caractérisé en ce que la teneur en Pd du catalyseur-support est de 0,2 à 5% en poids.
